Europäisches Patentamt

**European Patent Office**    (11) Publication number: **0196124**
**B1**

Office européen des brevets

## EUROPEAN PATENT SPECIFICATION

(45) Date of publication of the patent specification:
22.03.89

(51) Int. Cl.⁴: **C 07 C 1/04,** C 07 C 27/06,
C 07 C 29/15, C 07 C 31/04 //
B01J8/02

(21) Application number: 86200283.9

(22) Date of filing: 24.02.86

(54) Process for the preparation of organic compounds from synthesis gas.

(30) Priority: 28.03.85 NL 8500910

(73) Proprietor: SHELL INTERNATIONALE RESEARCH MAATSCHAPPIJ B.V., Carel van Bylandtlaan 30, NL-2596 HR Den Haag (NL)

(43) Date of publication of application:
01.10.86 Bulletin 86/40

(72) Inventor: van der Burgt, Maarten Johannes, Carel van Bylandtlaan 30, NL-2596 HR The Hague (NL)

(45) Publication of the grant of the patent:
22.03.89 Bulletin 89/12

(74) Representative: Aalbers, Onno et al, P.O. Box 302, NL-2501 CH The Hague (NL)

(84) Designated Contracting States:
DE FR GB IT NL

(56) References cited:
DE-B- 1 668 390
GB-A- 2 077 136
US-A- 4 215 011
US-A- 4 276 265
US-A- 4 289 855

CATALYSIS - SCIENCE AND TECHNOLOGY, vol. 1, 1981, Springer-Verlag, Berlin, Heidelberg, pp. 162-164

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The invention relates to a process for the preparation of organic compounds by passing feed gas in at least one reaction zone through fluid-permeable holders containing catalyst particles.

Such a process is known from US-A-4,215,011 employing catalyst particles which also serve as distillation packing; said particles are contained in a plurality of closed cloth pockets present in a spirally wound cloth belt or in cages of wire cloth supported by a save tray. The known process has as disadvantages the restriction of vapour flow through the cloth and attrition of the catalyst particles.

In the present process, in which synthesis gas is led continuously or discontinuously through a catalyst bed, it is often desirable to employ as small catalyst particles as possible in order to improve the yield per unit weight of catalyst. It is not, however, possible to reduce the particle size of the catalyst indefinitely, since the pressure drop in a catalyst bed in the direction of flow of the synthesis gas in greater as the catalyst particles are smaller. The greater pressure drop often cancels out the advantage ensuing from the employment of smaller particles.

It has now been found that the drawbacks connected with the employment of smaller catalyst particles packed in cages of wire cloth can largely be overcome by employing catalyst particles packed in stacked catalyst holders permeable to fluid (i.e. gas and liquid).

The invention therefore relates to a process for the preparation of organic compounds by passing feed gas in at least one reaction zone trough fluid-permeable holders containing catalyst particles, characterized in that the catalyst holders are stacked in the reaction zone(s) and are provided with a plurality of openings having a diameter of 0.05 to 0.7 mm and in that synthesis gas is employed as feed gas.

The process according to the invention causes only a relatively small pressure drop while the yield per unit weight of catalyst remains almost the same as that obtained by employing similar catalyst particles without said holders; in addition mass transport limitations arising from the employment of larger catalyst particles are largely eliminated. Moreover, heat transfer is improved.

The process according to the invention can be carried out continuously or discontinuously; preferably a continuous process is employed.

The particle size of the catalyst is preferably between 0.1 and 1.5 mm and more preferably between 0.25 and 1.0 mm, always based on the largest diameter of each particle.

The catalyst holder can in principle have any shape. Preferably, the height and width of the catalyst holder do not differ by more than 50% from the length; in particular, the catalyst holder has a regular shape, for example substantially cylindrical or spherical. Substantially spherical catalyst holder are the most preferred. The diameter of the catalyst holder is advantageously between 2 and 10 mm and in particular between 4 and 8 mm, always based on the largest diameter of each holder.

The catalyst holder suitably consists of a continuous network of material which, under the conditions under which the synthesis gas is contacted with the catalyst, is inert. Preferably, the catalyst holder consists of a closed network of aluminium or stainless steel.

Advantageously 20-95% and preferably 40-90% of the surface area of the holder consists of a permeable area. The permeable area is formed by a plurality of openings in e.g. a network, which openings have a diameter of 0.05 to 0.7 mm and in particular of 0.1 to 0,5 mm, the diameter of course always being chosen such that the catalyst particles in the catalyst holder cannot pass through the openings in the catalyst holder.

At least 50%, preferably at least 80% and in particular the entire volume of the reaction zone is occupied by the stacked catalyst holders forming interstices, the interstices between the stacked catalyst holders preferably occupying between 20 and 50% of the volume in the reaction zone.

The process according to the invention is in principle suitable for any conversion of synthesis gas into organic compounds. In such a conversion, the synthesis gas is passed at a particular temperature and pressure and with a particular space velocity through a catalyst bed containing a particular catalyst packed in the above-described catalyst holders. The choice of catalyst, temperature, pressure and space velocity depends on the desired product.

The process according to the invention is particularly suitable for the preparation of hydrocarbons and/or oxygen-containing hydrocarbon derivatives, such as methanol, from synthesis gas.

If the process according to the invention is carried out for the preparation of methanol, catalysts known in the art for converting synthesis gas into methanol should be employed as catalyst, e.g. a catalyst containing zinc together with chromium.

If the process according to the invention is carried out for the preparation of hydrocarbons, Fischer-Tropsch catalysts known in the art, which contain Fe, Co, Ru and/or Ni, can be employed. The present invention is suitable for the preparation of middle distillates. In this context «middle distillates» are understood to be hydrocarbon mixtures whose boiling range corresponds substantially to that of the kerosine and gas oil fractions, which are obtained in the classical atmospheric distillation of crude petroleum. The middle distillate range extends substantially from about 150 to 360°C.

Middle distillates can be prepared in one or two stages. They are prepared in one stage by passing the synthesis gas over a Fischer-Tropsch catalyst with one or more promotors and a carrier material. The products which can be prepared with these catalysts generally contain a very wide molecular weight distribution and, in addition to branched and non-branched paraffins, often contain considerable quantities of olefins and oxygen-containing organic compounds. The products obtained often contain only a small proportion of middle distillates. Besides the yield, the product characteristics of the gas oil obtained leave much to be desired as a consequence of the presence of the previously mentioned olefins and oxygen-containing organic compounds. That is why the two-stage method of preparation is preferably used, in which in a first step a class of Fischer-Tropsch catalysts is used, which catalysts have the property that they give a product containing only very small amounts of olefins and oxygen-containing organic compounds and consisting almost exclusively of non-branched paraffins, a large proportion of which boil above the middle distillate range, and in which in a second stage the high-boiling part of the product obtained in the first stage is converted by hydrocracking into middle distillates. As feedstock for the hydrocracking, at least that part of the product is chosen whose initial boiling point lies above the final boiling point of the heaviest middle distillate desired as end product. The hydrocracking, which is characterized by a low hydrogen consumption, gives middle distillates with a substantially better pour point than those obtained from the direct conversion of a $H_2/CO$ mixture according to the Fischer-Tropsch method.

The process according to the invention is advantageously employed in the first stage of this two-stage method of preparation.

The Fischer-Tropsch catalysts used in the first stage of the two-stage method of preparation preferably contain silica, alumina or silica-alumina as carrier material, and cobalt together with zirconium, titanium and/or chromium as catalytically active metals, preferably in such quantities that 3-60 parts by weight cobalt and 0.1-100 parts by weight zirconium, titanium and/or chromium are present per 100 parts by weight carrier material. The catalysts are advantageously prepared by kneading and/or impregnating the metals in question onto the carrier material. Before being used, the cobalt catalysts are preferably activated. This activating can be suitably carried out by contacting the catalyst at a temperature of between 200 and 350°C with hydrogen or a hydrogen-containing gas.

The conversion of synthesis gas into middle distillates is preferably carried out at a temperature of 125-350°C and in particular of 175-275°C and a pressure of 5-100 bar and in particular of 10-75 bar.

The $H_2$- and CO-containing feedstock, which is converted into middle distillates with the aid of the above-mentioned catalysts, preferably has an $H_2/CO$ mol. ratio of 1.0-2.5 and in particular of 1.5-2.25.

Although in the preparation of middle distillates from the product obtained via the synthesis catalyst, that part of the product whose initial boiling point lies above the final boiling point of the heaviest middle distillate desired as end product can be used as feedstock for hydrocracking, use is preferably made for this purpose of the total $C_9+$ fraction and in particular of the total $C_5+$ fraction of the product of the first stage, since it has been observed that under the influence of the catalytic hydrogen treatment, the quality of the gasoline, kerosine and gas oil fractions contained therein is improved.

The hydrocracking is carried out by contacting the fraction to be treated, at elevated temperature and pressure and in the presence of hydrogen, with a catalyst containing one or more noble metals from Group VIII on a carrier. As hydrocracking catalyst, a catalyst is preferably employed which contains 0.1-2 wt% and in particular 0.2-1 wt% of one or more noble metals from Group VIII on a carrier. Preferred catalysts are those which contain platinum or palladium as noble metal from Group VIII and silica-alumina as carrier. The hydrocracking is preferably carried out at a temperature of 200-400°C and in particular of 250-350°C and at a pressure of 5-10 bar and in particular of 10-75 bar.

If the reaction product prepared via the synthesis catalyst still contains sufficient unconverted hydrogen for carriyng out the hydrocracking, both steps can be carried out in «series flow». In that case, the $C_8$– fraction or the $C_4$– fraction is not separated after the first stage, but only after the second stage. As is known, carrying out a multi-stage process in series flow means that the total reaction product from a given stage, without components being removed from it or added to it, is used as feedstock for the following stage, which is carried out substantially at the same pressure as the previous stage.

The invention will now be illustrated with the aid of the following Examples of which only Example 3 relates to the process according to the invention.

*Comparative Example 1*

Synthesis gas (molar ratio $H_2 : CO = 2$) was contacted at 230°C and 20 bar and at a space velocity of 900 l (STP)/l catalyst/hour with 8 kg catalyst (9 parts by weight cobalt and 6 parts by weight zirconium per 100 parts by weight $SiO_2$ as carrier) in a reactor with a volume of 13 l. The catalyst consisted of catalyst particles with a diameter of 0.38 mm.

*Comparative Example 2*

Example 1 was repeated, except that the diameter of the catalyst particles was now 2.4 mm.

*Example 3*

Example 1 was repeated, excepts that the catalyst particles were packed in catalyst holders. The catalyst holders were spherical, had a diameter of 6 mm and consisted of a network of stainless steel with openings of 0.3 mm and a permeable area of 75% of the network area. The reactor was, except for the interstices, entirely filled with catalyst holders. The quantity of catalyst was 5.2 kg.

In these 3 Examples the yield (expressed as kg $C_1+ \cdot$ kg catalyst $^{-1} \cdot h^{-1}$) the selectivity (the part of the $C_1+$ fraction that belonged to the $C_3+$ fraction) and the pressure drop were determined.

The results are given in Table 1 below.

TABLE 1

| Example | 1 (Comp.) | 2 (Comp.) | 3 |
|---|---|---|---|
| diameter of catalyst particles | 0.38 mm | 2.4 mm | 0.38 mm |
| catalyst holder | — | — | — |
| yield | 123 | 52 | 120 |
| selectivity $(C_3+/C_1+)$ | 87 | 67 | 83 |
| pressure drop (bar) | 10 | 0.25 | 0.04 |

## Claims

1. Process for the preparation of organic compounds by passing feed gas in at least one reaction zone through fluid-permeable holders containing catalyst particles, characterized in that the catalyst holders are stacked in the reaction zone(s) and are provided with a plurality of openings having a diameter of 0.05 to 0.7 mm and in that synthesis gas is employed as feed gas.

2. Process according to claim 1 wherein the catalyst particles have a diameter lying between 0.1 and 1.5 mm.

3. Process according to claim 1 or 2 wherein the catalyst holders have a width and height not differing by more than 50% from the length.

4. Process according to any of the preceding claims wherein the catalyst holders are substantially cylindrical or spherical.

5. Process according to any of the preceding claims wherein the catalyst holders have a diameter lying between 2 and 10 mm.

6. Process according to any of the preceding claims wherein the interstices between the stacked catalyst holders occupy between 20 and 50% of the volume in the reaction zone.

7. Process according to any of the preceding claims wherein the catalyst holders consist of a continuous network of material which is inert under the conditions under which the synthesis gas is contacted with the catalyst.

8. Process according to claim 7 wherein the catalyst holders consist of a closed network of aluminium or stainless steel.

9. Process according to any of the preceding claims wherein between 20 and 95% of the surface area of the holders consists of permeable area.

10. Process according to any of the preceding claims wherein the holders are provided with openings having a diameter lying between 0.1 and 0.5 mm.

## Patentansprüche

1. Verfahren zur Herstellung von organischen Verbindungen mittels Einleiten eines Zuspeisungsgases in mindestens eine Reaktionszone durch fluiddurchlässige, Katalysatorteilchen enthaltende Trägers (holders), dadurch gekennzeichnet, daß die Katalysatorträger in der (den) Reaktionszonen gestapelt vorliegen und mit einer Vielzahl von Öffnungen mit einem Durchmesser von 0,05 bis 0,7 mm versehen sind und daß ein Synthesegas als Zuspeisungsgas verwendet wird.

2. Verfahren nach Anspruch 1, in welchem die Katalysatorteilchen einen Durchmesser zwischen 0,1 und 1,5 mm aufweisen.

3. Verfahren nach Anspruch 1 oder 2, in welchem die Katalysatorträger eine Breite und eine Höhe aufweisen, die nicht mehr als 50% von der Länge abweichen.

4. Verfahren nach einem der vorstehenden Ansprüche, in welchem die Katalysatorträger im wesentlichen zylindrische oder kugelförmige Gestalt besitzen.

5. Verfahren nach einem der vorstehenden Ansprüche, in welchem die Katalysatorträger einen Durchmesser zwischen 2 und 10 mm aufweisen.

6. Verfahren nach einem der vorstehenden Ansprüche, in welchem die Zwischenräume zwischen den gestapelten Katalysatorträgern zwischen 20 und 50% des Volumens der Reaktionszone einnehmen.

7. Verfahren nach einem der vorstehenden Ansprüche, in welchem die Katalysatorträger aus einem kontinuierlichen Materialnetz bestehen, welches unter den bei Kontaktierung des Synthesegases mit dem Katalysator herrschenden Bedingungen inert ist.

8. Verfahren nach Anspruch 7, in welchem die Katalysatorträger aus einem geschlossenen Netz aus Aluminium oder Edelstahl bestehen.

9. Verfahren nach einem der vorstehenden Ansprüche, in welchem zwischen 20 und 95% der Oberfläche der Träger eine durchlässige Fläche darstellen.

10. Verfahren nach einem der vorstehenden Ansprüche, in welchem die Träger mit Öffnungen mit einem Durchmesser zwischen 0,1 und 0,5 mm versehen sind.

## Revendications

1. Procédé de préparation de composés organiques en faisant passer du gaz d'alimentation dans au moins une zone de réaction à travers des récipients perméables aux fluides contenant des particules de catalyseur, caractérisé en ce que les récipients de catalyseur sont empilés dans la (les) zone(s) de réaction et sont munis de plusieurs ouvertures ayant un diamètre de 0,05 à 0,7 mm et en ce qu'on emploie du gaz de synthèse comme gaz d'alimentation.

2. Procédé selon la revendication 1 où les particules de catalyseur ont un diamètre situé entre 0,1 et 1,5 mm.

3. Procédé selon la revendication 1 ou 2 où les récipients de catalyseur ont une largeur et une hauteur ne différant pas de plus de 50% de la longueur.

4. Procédé selon l'une quelconque des revendications précédentes dans lequel les récipients de catalyseur sont essentiellement cylindriques ou sphériques.

5. Procédé selon l'une quelconque des revendications précédentes dans lequel les récipients de catalyseur ont un diamètre situé entre 2 et 10 mm.

6. Procédé selon l'une quelconque des revendications précédentes dans lequel les interstices entre les récipients de catalyseur empilés occupent entre 20 et 50% du volume dans la zone de réaction.

7. Procédé selon l'une quelconque des revendications précédentes dans lequel les récipients de catalyseur consistent en un réseau continu de matière qui est inerte dans les conditions dans lesquelles le gaz de synthèse est mis en contact avec le catalyseur.

8. Procédé selon la revendication 7 dans lequel les récipients de catalyseur consistent en un réseau fermé d'aluminium ou d'acier inoxydable.

9. Procédé selon l'une quelconque des revendications précédentes dans lequel entre 20 et 95% de la surface des récipients consiste en une surface perméable.

10. Procédé selon l'une quelconque des revendications précédentes dans lequel les récipients sont munis d'ouvertures ayant un diamètre situé entre 0,1 et 0,5 m.